(19) Europäisches Patentamt
European Patent Office
Office européen des brevets

(11) EP 2 508 071 A1

(12) EUROPEAN PATENT APPLICATION

(43) Date of publication:
10.10.2012 Bulletin 2012/41

(51) Int Cl.:
*A01N 27/00* (2006.01) *A01N 25/18* (2006.01)
*A01N 25/08* (2006.01)

(21) Application number: 12159293.5

(22) Date of filing: 13.03.2012

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 05.04.2011 US 201161471786 P

(71) Applicant: **Rohm and Haas Company**
**Philadelphia, Pennsylvania 19106-2399 (US)**

(72) Inventor: **Jacobson, Richard, Martin**
**Chalfont, PA 18914 (US)**

(74) Representative: **Buckley, Guy Julian**
**Patent Outsourcing Limited**
**1 King Street**
**Bakewell**
**Derbyshire DE45 1DZ (GB)**

# (54) Controlled release compositions

(57) A composition comprising (a) a meltable solid matrix comprising (i) one or more solid hydrophobic substance, and (ii) one or more solid hydrophilic substance, and (b) distributed in said matrix, one or more encapsulation complex of a volatile cyclopropene compound encapsulated in a molecular encapsulation agent. Also provided is a method of treating plants or plant parts comprising placing said plants or plant parts and the composition of claim 1 into a container and allowing said plants or plant parts and said composition of claim 1 to remain together in said container for 1 day or longer.

EP 2 508 071 A1

## Description

Handling volatile compounds presents a variety of problems. One method of making a volatile compound easier to handle is to form a molecular encapsulation complex in which a molecular encapsulating agent encapsulates the molecules of the volatile compound. In some cases, the molecular encapsulation complex is in the form of a powder, which may optionally be blended with other solid particles of other materials to form a blended powder. Such powders are generally easier to store, to transport, and/or to use than the pure volatile compound.

When it is desired to make use of the volatile compound that is contained in such a powder, one common method involves bringing the powder into contact with a release compound, which is a compound that, when it contacts an encapsulation complex, promotes or causes release of the volatile compound from the molecular encapsulation complex. For some useful molecular encapsulation complexes, water is a release compound.

However, providing a molecular encapsulation complex as a powder does not solve all the problems associated with the use of the volatile compounds. For example, when using a release compound to release the volatile compound from such a powder, the contact between the powder and the release compound may not be intimate, and the release of the volatile compound may be incomplete or undesirably slow or both. In such cases it may be necessary to take some measure to enhance the release of the volatile compound, such as, for example, waiting an undesirably long time for the volatile compound to release; providing some mechanical aid (e.g., shaking, stirring, forcing gas into the water to create bubbles, etc.); or providing additives to the powder that cause effervescent action on contact with water. U.S. Patent No. 6,426,319 describes a composition containing a molecular encapsulation complex of a molecular encapsulating agent with a cyclopropene compound, mixed with a water absorbent material.

In many situations, it is desired that the volatile compound is released gradually when the composition containing the volatile compound is exposed to a release compound. However, compositions like those of U.S. Patent No. 6,426,319 generally release the cyclopropene compound relatively quickly when exposed to water vapor.

It is desired to provide a composition that contains a useful volatile compound in a molecular encapsulation complex and that releases that useful volatile compound desirably gradually when exposed to liquid water or to water vapor.

The following is a statement of the invention.

The first aspect of the present invention is a composition comprising (a) a meltable solid matrix comprising (i) one or more solid hydrophobic substance, and (ii) one or more solid hydrophilic substance, and (b) distributed in said matrix, one or more encapsulation complex of a volatile cyclopropene compound encapsulated in a molecular encapsulation agent.

Another aspect of the present invention is a method of treating plants or plant parts comprising placing said plants or plant parts and the composition of the first aspect into a container and allowing said plants or plant parts and said composition of claim 1 to remain together in said container for 1 day or longer.

The following is a detailed description of the invention.

As used herein, the following terms have the designated definitions, unless the context clearly indicates otherwise.

As used herein, "hydrocarbon" refers to a chemical group or a compound, the atoms of which include one or more carbon atom, one or more hydrogen atom, and no other atoms.

As used herein, a "matrix" is a material that is a solid and that has continuity. In a macroscopic sample of a composition that contains a matrix, the matrix will form a continuous 3-dimensional body. The matrix may connect with the entire surface of the sample; if it does not connect with the entire surface of the sample, it will connect to multiple places on the surface of the sample. At least 50% of the total surface area of the sample will connect to the matrix. The matrix may be a pure material or may be a mixture of two or more materials.

A macroscopic sample is a body that has volume of at least one cubic centimeter and has a shortest axis of 3 mm or longer. An axis is a line segment that passes through the center of mass of the body and that has end points that reside on the surface of the body.

If the matrix is a mixture, the ingredients in the mixture will form either a solution or a dispersion or a combination thereof. In a solution, the ingredients are miscible with each other; that is, they will be dissolved in each other (i.e., intimately mixed on the molecular level). In a dispersion, one ingredient (or a solution of ingredients) is present as a continuous phase, and one or more other ingredients is present as small particles (1 millimeter in diameter or smaller) that are distributed throughout the continuous phase.

A particle (whether in a powder or dispersed in a matrix) is characterized by its diameter. If the particle is not spherical, the diameter of the particle is considered herein to be the diameter of a sphere that has the same volume as the particle.

As used herein, material is said to be solid if it is in the solid state over a range of temperatures that includes at least 0°C to 40°C. Solids include, for example, crystalline solids, amorphous glassy solids, waxy solids, and rubbery solids. A macroscopic sample of a solid will not deform appreciably under its own weight. A cube of a solid material that

is 1 cm X 1 cm X 1 cm in size; and that is placed on a flat surface at 25°C for 7 days, will maintain at least 95 % of its height.

As used herein, a material is said to be a meltable solid if it is a solid as defined herein above and if it also has a melting point or a softening point higher than 40°C and lower than 120°C. The softening point is a temperature above which a material is liquid or, if not liquid, is soft enough to be blended or masticated with a mechanical device such as, for example, a stirrer, an extruder, or a kneader.

A compound is said herein to be volatile if that compound has a boiling point at 1 atmosphere pressure of 100°C or lower.

As used herein, a hydrophobic substance is a substance that meets one or more of the following hydrophobicity criteria. The first hydrophobicity criterion is the water contact angle. A substance is hydrophobic if a clean surface of that substance has contact angle with a drop of water of 90° or higher. The contact angle is measured by test D7334-08 (ASTM International, West Conshohocken, PA, USA).

The second hydrophobicity criterion is the composition. A substance is hydrophobic if it is a pure hydrophobic substance or if it is a hydrophobic mixture. A hydrophobic mixture contains one or more pure hydrophobic substance, and the amount of all the pure hydrophobic substances in the mixture is 75% or more by weight, based on the weight of the mixture. A substance is a pure hydrophobic substance if its molecule has one or more fatty groups and if it has solubility in water at 25°C of less than 1% by weight based on the weight of the water. A fatty group is one of the following: (1) an aliphatic group that has 8 or more carbon atoms; that has only hydrogen atoms and carbon atoms; that is linear, branched, cyclic, or a combination thereof; (2) a chemical group of category (1) in which one or more hydrogen atom is replaced with a halogen atom; or (3) a silicone group. A silicone group is a group that contains a linear chain of 5 or more siloxane groups. A siloxane group has the following structure (I):

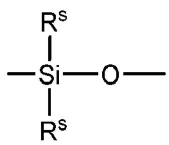

(I)

where each $R^s$ is, independent of every other $R^s$, hydrogen, a hydrocarbon group with 6 or fewer carbon atoms, or another siloxane group.

The third hydrophobicity criterion is water absorptivity. When a hydrophobic substance is in equilibrium at 25°C with air having 100% relative humidity, the hydrophobic substance will absorb water from the air in an amount such that the quotient of the weight of absorbed water divided by the dry weight of hydrophilic substance is 0.0001 or lower.

The fourth hydrophobicity criterion is water vapor transmission rate. When a film of a hydrophobic material that is 40 micrometers thick is tested at 37.8°C and 90% relative humidity, its water vapor transmission rate is less than 25 grams of water vapor per square meter of film per day.

The fifth hydrophobicity criterion is as follows: a polymer that is a copolymer of ethylene and vinyl acetate is hydrophobic if the amount of vinyl acetate in that copolymer is less than 20% by weight, based on the weight of the copolymer.

As used herein, a hydrophilic substance is a substance that meets one or both of the following hydrophilicity criteria. The first hydrophilicity criterion is water absorptivity: when a hydrophilic substance is in equilibrium at 25°C with air having 100% relative humidity, the hydrophilic substance will absorb water from the air in an amount such that the quotient of the weight of absorbed water divided by the dry weight of hydrophilic substance is 0.001 or higher.

The second hydrophilicity criterion is water vapor transmission rate. When a film of a hydrophilic material that is 40 micrometers thick is tested at 37.8°C and 90% relative humidity, its water vapor transmission rate is more than 50 grams of water vapor per square meter of film per day.

The third hydrophilicity criterion is as follows: a polymer that is a copolymer of ethylene and vinyl acetate is hydrophilic if the amount of vinyl acetate in that copolymer is 20% or more by weight, based on the weight of the copolymer.

As used herein, a humectant is a substance that, when placed in contact with air of 100% relative humidity at 25°C, will absorb water from the air. When a humectant reaches equilibrium with air of 100% relative humidity at 25°C, the quotient of the weight of water absorbed by the humectant divided by the dry weight of the humectant is 0.1 or higher. Some humectants are deliquescent substances. A deliquescent substance, in equilibrium at 25°C with air having 100% relative humidity, will absorb sufficient water from the air to form a liquid solution of the deliquescent substance in water.

As used herein, a water-adsorptive substance is a solid material that has pores, tunnels, or other cavities that have the capacity to trap water molecules. The molecules of a water-adsorptive substance do not swell or otherwise re-arrange in response to the presence of an adsorbed water molecule. Prior to exposure to water, the cavities are empty (i.e., each cavity contains only air or vacuum). A water molecule that enters such a cavity and remains there for a finite time period is known herein as "adsorbed" water.

When a molecular encapsulation agent encapsulates a compound or a portion of a compound, the resulting

combination is referred to herein as a molecular encapsulation complex.

A "polymer," as used herein and as defined by FW Billmeyer, JR. in Textbook of Polymer Science, second edition, 1971, is a relatively large molecule made up of the reaction products of smaller chemical repeat units. Polymers may have structures that are linear, branched, star shaped, looped, hyperbranched, crosslinked, or a combination thereof; polymers may have a single type of repeat unit ("homopolymers") or they may have more than one type of repeat unit ("copolymers"). Copolymers may have the various types of repeat units arranged randomly, in sequence, in blocks, in other arrangements, or in any mixture or combination thereof. A compound that reacts with identical and/or non-identical compounds to become a repeat unit in a polymer is known herein as a "monomer." The repeat unit that results from such a reaction is known herein as a "residue" of that monomer.

Polymer molecular weights can be measured by standard methods such as, for example, size exclusion chromatography (SEC, also called gel permeation chromatography or GPC). Polymers have number-average molecular weight (Mw) of 500 or more.

Polymers are crosslinked if some or all of the polymer chains are branched. A polymer is lightly crosslinked if sufficient branching is present to affect the physical properties of the polymer and the polymer has finite molecular weight. A polymer is fully crosslinked if sufficient branching is present to pass the percolation threshold. A fully crosslinked polymer is not soluble in any solvent, even a solvent in which a linear polymer of the same composition is soluble. A fully crosslinked polymer is said to have infinite molecular weight.

One useful characterization of a composition is the solids content. The solids content of a composition is determined by making a layer of the composition that is 1 mm or less in thickness, allowing volatile compounds to leave the composition, for example by placing that layer in a oven that circulates air at 100°C for one hour. The weight of the remaining material after the volatiles have left, divided by the original weight of the composition, expressed as a percentage, is the solids content.

The present invention involves the use of one or more cyclopropene compound. As used herein a cyclopropene compound is any compound with the formula

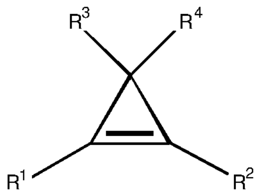

where each $R^1$, $R^2$, $R^3$ and $R^4$ is independently selected from the group consisting of H and a chemical group of the formula:

$$-(L)_n-Z$$

where n is an integer from 0 to 12. Each L is a bivalent radical. Suitable L groups include, for example, radicals containing one or more atoms selected from H, B, C, N, O, P, S, Si, or mixtures thereof. The atoms within an L group may be connected to each other by single bonds, double bonds, triple bonds, or mixtures thereof. Each L group may be linear, branched, cyclic, or a combination thereof. In any one R group (i.e., any one of $R^1$, $R^2$, $R^3$ and $R^4$) the total number of heteroatoms (i.e., atoms that are neither H nor C) is from 0 to 6.

Independently, in any one R group the total number of non-hydrogen atoms is 50 or less.

Each Z is a monovalent radical. Each Z is independently selected from the group consisting of hydrogen, halo, cyano, nitro, nitroso, azido, chlorate, bromate, iodate, isocyanato, isocyanido, isothiocyanato, pentafluorothio, and a chemical group G, wherein G is a 3 to 14 membered ring system.

The $R^1$, $R^2$, $R^3$, and $R^4$ groups are independently selected from the suitable groups. The $R^1$, $R^2$, $R^3$, and $R^4$ groups may be the same as each other, or any number of them may be different from the others. Groups that are suitable for use as one or more of $R^1$, $R^2$, $R^3$, and $R^4$ may be connected directly to the cyclopropene ring or may be connected to the cyclopropene ring through an intervening group such as, for example, a heteroatom-containing group.

As used herein, a chemical group of interest is said to be "substituted" if one or more hydrogen atoms of the chemical group of interest is replaced by a substituent. Suitable substituents include, for example, alkyl, alkenyl, acetylamino, alkoxy, alkoxyalkoxy, alkoxycarbonyl, alkoxyimino, carboxy, halo, haloalkoxy, hydroxy, alkylsulfonyl, alkylthio, trialkylsilyl, dialkylamino, and combinations thereof.

Among the suitable $R^1$, $R^2$, $R^3$, and $R^4$ groups are, for example, substituted and unsubstituted versions of any one of the following groups: aliphatic, aliphatic-oxy, alkylcarbonyl, alkylphosphonato, alkylphosphato, alkylamino, alkylsulfonyl, alkylcarboxyl, alkylaminosulfonyl, cycloalkylsulfonyl, cycloalkylamino, heterocyclyl (i.e., aromatic or non-aromatic cyclic groups with at least one heteroatom in the ring), aryl, hydrogen, fluoro, chloro, bromo, iodo, cyano, nitro,

nitroso, azido, chlorato, bromato, iodato, isocyanato, isocyanido, isothiocyanato, pentafluorothio; acetoxy, carboethoxy, cyanato, nitrato, nitrito, perchlorato, allenyl; butylmercapto, diethylphosphonato, dimethylphenylsilyl, isoquinolyl, mercapto, naphthyl, phenoxy, phenyl, piperidino, pyridyl, quinolyl, triethylsilyl, and trimethylsilyl.

Among the suitable $R^1$, $R^2$, $R^3$, and $R^4$ groups are those that contain one or more ionizable substituent groups. Such ionizable groups may be in non-ionized form or in salt form.

Also contemplated are embodiments in which $R^3$ and $R^4$ are combined into a single group, which is attached to the number 3 carbon atom of the cyclopropene ring by a double bond. Some of such compounds are described in US Patent Publication 2005/0288189.

In preferred embodiments, one or more cyclopropene compound is used in which each of $R^1$, $R^2$, $R^3$, and $R^4$ is independently hydrogen or a substituted or unsubstituted alkyl, alkenyl, alkynyl, cycloalkyl, cycloalkylalkyl, phenyl, or naphthyl group; where the substituents, when present, are independently halogen, alkoxy, or substituted or unsubstituted phenoxy. In more preferred embodiments, one or more of $R^1$, $R^2$, $R^3$, and $R^4$ is hydrogen and each of $R^1$, $R^2$, $R^3$, and $R^4$ that is not hydrogen is independently a substituted or unsubstituted alkyl, alkenyl, alkynyl, cycloalkyl, cycloalkylalkyl, phenyl, or naphthyl group; where the substituents, when present, are independently halogen, alkoxy, or substituted or unsubstituted phenoxy. In more preferred embodiments, each of $R^2$, $R^3$, and $R^4$ is hydrogen, and $R^1$ is hydrogen or an unsubstituted alkyl, alkenyl, alkynyl, cycloalkyl, cycloalkylalkyl, phenyl, or naphthyl group independently.

In preferred embodiments, one or more cyclopropene compound is used in which one or more of $R^1$, $R^2$, $R^3$, and $R^4$ is hydrogen each of $R^1$, $R^2$, $R^3$, and $R^4$ that is not hydrogen is (C1-C4) alkyl. In more preferred embodiments, $R^1$ is (C1-C4) alkyl and each of $R^2$, $R^3$, and $R^4$ is hydrogen. In more preferred embodiments, $R^1$ is methyl and each of $R^2$, $R^3$, and $R^4$ is hydrogen, and the cyclopropene compound is known herein as "1-MCP."

In preferred embodiments, a cyclopropene compound is used that has boiling point at one atmosphere pressure of 25°C or lower; more preferred is 15°C or lower. Independently, in preferred embodiments, a cyclopropene compound is used that has boiling point at one atmosphere pressure of -100°C or higher; more preferred is -50°C or higher; more preferred is-25°C or higher; more preferred is 0°C or higher.

The composition of the present invention includes at least one molecular encapsulating agent that encapsulates one or more cyclopropene compound or a portion of one or more cyclopropene compound. A complex that contains a cyclopropene compound molecule or a portion of a cyclopropene compound molecule encapsulated in a molecule of a molecular encapsulating agent is known herein as a "cyclopropene compound complex."

In preferred embodiments, at least one cyclopropene compound complex is present that is an inclusion complex. In such an inclusion complex, the molecular encapsulating agent forms a cavity, and the cyclopropene compound or a portion of the cyclopropene compound is located within that cavity.

Preferably, in such inclusion complexes, the interior of the cavity of the molecular encapsulating agent is substantially apolar or water-incompatible or both, and the cyclopropene compound (or the portion of the cyclopropene compound located within that cavity) is also substantially apolar or water-incompatible or both. While the present invention is not limited to any particular theory or mechanism, it is contemplated that, in such apolar cyclopropene compound complexes, van der Waals forces, or water-incompatibility interactions, or both, cause the cyclopropene compound molecule or portion thereof to remain for substantial amounts of time within the cavity of the molecular encapsulating agent.

The amount of molecular encapsulating agent can usefully be characterized by the quotient ("Q1") of the number of moles of molecular encapsulating agent divided by the number of moles of cyclopropene compound. In preferred embodiments, Q1 is 0.1 or larger; more preferably 0.2 or larger; more preferably 0.5 or larger; more preferably 0.9 or larger. Independently, in preferred embodiments, Q1 is 10 or lower; more preferably 5 or lower; more preferably 2 or lower; more preferably 1.5 or lower.

Suitable molecular encapsulating agents include, for example, organic and inorganic molecular encapsulating agents. Preferred are organic molecular encapsulating agents, which include, for example, substituted cyclodextrins, unsubstituted cyclodextrins, and crown ethers. Suitable inorganic molecular encapsulating agents include, for example, zeolites. Mixtures of suitable molecular encapsulating agents are also suitable. In preferred embodiments, the encapsulating agent is alpha cyclodextrin, beta cyclodextrin, gamma cyclodextrin, or a mixture thereof. In more preferred embodiments of the invention, alpha cyclodextrin is used.

The present invention involves the use of one or more solid hydrophobic substance (i) (herein referred to synonymously as "hydrophobic substance (i)" or "substance (i)"). Preferred hydrophobic substances (i) are fatty compounds, hydrocarbon waxes, olefin polymers, and mixtures thereof.

A fatty compound is a compound that has one or more fatty group. Fatty compounds include, for example, fatty acids, fatty oils, modified versions thereof, and mixtures thereof. Suitable modifications include any process, including chemical reactions, that alters the composition of a fatty compound, as long as the resulting compound still meets the definition of fatty compound. Modifications include, for example, hydrogenation, esterification, trans-esterification, de-esterification, polymerization, attachment of functional groups, and combinations thereof. Fatty acids have the formula R-COOH, where the R group contains a fatty group. Fatty oils are fatty compounds that contain one or more ester group, hydroxyl group, aldehyde group, ketone group, or combination thereof.

Among fatty acids, preferred are those that include at least one fatty group having 12 or more carbon atoms. More preferred are fatty acids that include at least one fatty group having 16 or more carbon atoms; more preferred are fatty acids that include at least one fatty group having 18 or more carbon atoms. Preferred fatty acids are those that include at least one fatty group having 22 or fewer carbon atoms. Preferred are fatty acids that include at least one fatty group that is a hydrocarbon group that is saturated (i.e., within that group, the bonds between carbon atoms are all single bonds). Among fatty acids, more preferred is stearic acid, palmitic acid, and mixtures thereof. Among fatty acids, more preferred is double pressed stearic acid, which is a mixture of fatty acids in which a mixture of stearic and palmitic acids comprises 93% to 100% by weight, based on the weight of the double pressed stearic acid.

Also among the suitable hydrophobic substances are fatty oils. Preferred among fatty oils are triglycerides. Triglycerides are triesters of glycerol with three fatty acids. Preferred triglycerides are hydrogenated vegetable oils.

Hydrocarbon waxes are hydrocarbon compounds having 12 to 120 carbon atoms. A hydrocarbon molecule in a hydrocarbon wax may be straight, branched, non-aromatic cyclic, or a combination thereof. Hydrocarbon waxes usually exist as a mixture of 2 or more different hydrocarbon molecules. Hydrocarbon waxes include petroleum waxes, which are separated from crude petroleum. Petroleum waxes include paraffin wax, microcrystalline wax, and petrolatum. Paraffin wax has 60% or more by weight straight-chain hydrocarbons, based on the weight of the paraffin, and most of the straight-chain hydrocarbons in paraffin wax typically have 18 to 45 carbon atoms each. Microcrystalline wax has a higher proportion of branched and cyclic hydrocarbons than paraffin wax. Petrolatum is a type of microcrystalline wax that blends well with mineral oil. Among hydrocarbon waxes, preferred are microcrystalline waxes.

Olefin polymers are polymers, the repeat units of which are 60% to 100% by weight, based on the weight of the olefin polymer, residues of one or more ethylenically unsaturated hydrocarbon monomer. Any olefin polymer that meets the definition of hydrophobic substance (i) is suitable as hydrophobic substance (i). All-olefin polymers, which are olefin polymers in which the repeat units are 100% residues of one or more ethylenically unsaturated hydrocarbon monomer, are suitable as hydrophobic substance (i). Polyolefin waxes, which are all-olefin polymers that have number-average molecular weight of 20,000 or lower, are also suitable as hydrophobic substance (i).

Olefin polymers also include olefin copolymers, which are olefin polymers in which one or more repeat unit is a residue of a monomer that is not a hydrocarbon. A monomer that is not a hydrocarbon and that is capable of copolymerizing with one or more ethylenically unsaturated hydrocarbon monomer is known herein as an "olefin-compatible comonomer." Suitable olefin-compatible comonomers include, for example, acrylic acid, methacrylic acid, alkyl esters of acrylic acid or methacrylic acid, and vinyl acetate. Among olefin copolymers, preferred as hydrophobic substance (i) are copolymers of ethylene and vinyl acetate ("EVA copolymers") that meet the criteria for hydrophobicity as defined herein. Some preferred olefin copolymers for use as hydrophobic substance (i) are EVA copolymers in which the amount of residues of vinyl acetate is, by weight based on the weight of the EVA copolymer, 21% to 40%.

In preferred embodiments, the composition of the present invention contains one or more hydrophobic substance (i) that is a meltable solid. Among meltable solid hydrophobic substances, preferred are those with a melting point or a softening point that is 50°C or higher; more preferred is 60°C or higher. Among meltable solid hydrophobic substances, preferred are those with a melting point or a softening point that is 110°C or lower; more preferred is 90°C or lower; more preferred is 80°C or lower.

Preferred hydrophobic substances (i) contain, optionally among other ingredients, fatty compounds and hydrocarbon waxes and mixtures thereof. More preferred hydrophobic substances (i) contain, optionally among other ingredients, fatty acids, fatty oils, hydrocarbon waxes, and mixtures thereof. More preferred hydrophobic substances (i) contain, optionally among other ingredients, double pressed stearic acid, hydrogenated vegetable oils, microcrystalline waxes, and mixtures thereof.

The present invention involves the use of one or more hydrophilic substance (ii) (herein referred to synonymously as "hydrophilic substance (ii)" or "substance (ii)"). Preferred hydrophilic substances (ii) are polymers. Preferred polymers for hydrophilic substance (ii) include cellulose, polyethylene glycol, hydrophilic ethylene vinyl acetate copolymers, and pendant-amine polymers.

Cellulose is a polymer that made of repeat units of D-glucose. Among celluloses, preferred are natural cellulose, microcrystalline cellulose, and mixtures thereof.

Polyethylene glycol is a polymer made of repeat units of ethylene oxide:

$$-(CH_2-CH_2-O)-.$$

Among polyethylene glycols, preferred for use as hydrophilic substance (ii) are those with number-average molecular weight that is 20,000 or less; more preferred is 10,000 or less. Among polyethylene glycols, preferred for use as hydrophilic substance (ii) are those with number-average molecular weight that is more than 3,000.; more preferred is 5,000 or more.

A pendant-amide polymer is a polymer in which 50% or more of the residues, by weight, based on the weight of the pendant-amide polymer, has a chemical group that is pendant from the polymer backbone, where that pendant chemical group is attached to the polymer backbone by one or more covalent bond, and where that pendant chemical

group has an amide group (which may be all or part of the pendant chemical group). The amide group may be primary, secondary, or tertiary. Among pendant chemical groups, preferred is a 2-pyrrolidone group, preferably with the nitrogen atom attached by a covalent bond to a carbon atom on the polymer backbone. Among preferred pendant amide polymers, preferred are PVP polymers, which are polymers, the polymerized residues of which are 35% or more, by weight based on the weight of the PVP polymer, residues of N-vinyl-2-pyrrolidone. Among PVP polymers, preferred are crosslinked PVP polymers.

Preferred hydrophilic substances (ii) contain one of the following species, optionally with other ingredients: (A) one or more polyethylene glycol having number average molecular weight of more than 3,000, (B) one or more mixture of one or more polyethylene glycol having number average molecular weight of more than 3,000 with one or more cellulose, (C) one or more hydrophilic ethylene vinyl acetate copolymer, (D) one or more mixture of one or more hydrophilic ethylene vinyl acetate copolymer with one or more crosslinked PVP, or (E) one or more mixture of one or more hydrophilic ethylene vinyl acetate copolymer with one or more cellulose.

Some hydrophilic substances (ii) used in the present invention are hydrophilic substances that are also meltable solids. Among hydrophilic substances that are meltable solids, preferred are those with a melting point or a softening point that is 50°C or higher; more preferred is 60°C or higher. Preferred are hydrophilic substances with a melting point or a softening point that is 110°C or lower; more preferred is 90°C or lower; more preferred is 80°C or lower.

The composition of the present invention contains a meltable matrix that contains one or more hydrophobic substance (i) and one or more hydrophilic substance (ii). In some embodiments, the matrix is a solution of all of the hydrophobic substance(s) (i) and all of the hydrophilic substance(s) (ii). That is, all of the substance(s) (i) and (ii) are sufficiently soluble in each other that the mixture is a solution.

In preferred embodiments, the meltable matrix has a melting point higher than 40°C and lower than 120°C. Preferred is matrix melting point of 50°C or higher; more preferred is 60°C or higher. Preferred is matrix melting point of 110°C or lower; more preferred is 90°C or lower; more preferred is 80°C or lower. It is useful to characterize the viscosity (at shear rate of 1 $sec^{-1}$) of the matrix in the "pour range," which is the temperature range from 3 °C above the melting point to the lower of 125°C and 25 °C above the melting point. Preferably, the viscosity of the matrix in the pour range is 3,000 milliPascal*sec (3,000 centipoise) or lower; more preferably 1,000 milliPascal*sec (1,000 centipoise) or lower; more preferably 500 milliPascal*sec (500 cenitpoise) or lower.

In some embodiments ("dispersion embodiments"), in the matrix, one or more of the substance(s) (i) or one or more of the substance(s) (ii) is present as particles in a dispersion in the continuous phase of the matrix. In dispersion embodiments, the continuous phase of the matrix contains one or more hydrophobic substance (i). The amount of hydrophobic substance (i) found in the continuous phase of the matrix is characterized by the quotient ("Q2") found by dividing the sum of the weights of all the hydrophobic substances (i) contained in the continuous phase of the matrix by the total weight of the continuous phase of the matrix. Preferably, Q2 is 0.2 or higher; more preferably 0.3 or higher.

Preferred compositions of the present invention contain one or more humectant. Preferred humectants are potassium acetate and polyethylene glycols. Polyethylene glycols that are preferred for use as a humectant have number-average molecular weight that is 3,000 or less; more preferred is 2,000 or less. Polyethylene glycols that are preferred for use as a humectant have number-average molecular weight that is 500 or more. A polyethylene glycol is considered herein to be used as a humectant when at least one meltable solid hydrophilic substance (ii) that is not a polyethylene glycol is present in the composition.

In some embodiments, one or more other substances may be present in the composition. Such other substances do not qualify as either hydrophobic substance (i) or hydrophilic substance (ii) or as volatile cyclopropene compound encapsulated in a molecular encapsulation agent. Some other substances may be either hydrophobic or hydrophilic but are not meltable solids. Suitable other substances include, for example, dispersants, humectants, deliquescent salts, other salts, water-adsorptive materials, and mixtures thereof.

Preferred compositions of the present invention contain one or more other substance that is a water-adsorptive material. Preferred water-adsorptive materials are molecular sieves. Molecular sieves have open structures with uniform-size pores into which water molecules may enter and, possibly, remain. Molecular sieves may be made of aluminosilicates, clays, glasses, charcoals, or carbon. Preferred molecular sieves have pore size of 3 Angstrom or 4 Angstrom. Molecular sieves are supplied in the form of beads, pellets, or powder. In a sample of molecular sieves, average particle diameter of beads or pellets is normally 1mm to 5mm, and average particle diameter of powder is smaller than 1 mm. Molecular sieves are considered herein to be non-meltable solids. It is considered that molecular sieves, when present, are dispersed in the matrix.

Dispersants are amphiphilic compounds. One portion of the dispersant molecule is more stable when in contact with hydrophobic substance than it is when in contact with hydrophilic substance, and a different portion of the dispersant molecule is more stable when in contact with hydrophilic substance than it is when in contact with hydrophobic substance. For example, it may be desirable to use one or more dispersant when it is desired to disperse one or more hydrophilic substance (ii) in a continuous phase of one or more hydrophobic substance (i). For another example, it may be desirable to use one or more dispersant when it is desired to disperse powder particles of an encapsulated complex in a matrix,

for example when one portion of the dispersant molecule is more stable when in contact with the powder particles than it is when in contact with the matrix, and a different portion of the dispersant molecule is more stable when in contact with the matrix than it is when in contact with the powder particles.

Among embodiments in which one or more wax is used, it is preferred to also use one or more dispersant.

Preferred are compositions that contain one or more high-melting humectants (i.e., humectants that have melting point higher than 120°C). Preferred high-melting humectants are inorganic salts. More preferred is potassium acetate.

Some suitable high-melting humectants are inorganic salts that are also deliquescent.

The matrix of the present invention may be characterized by the hydrophilic index ("HI"), which is defined as follows:

$$HI = 20 \times (H / T)$$

where H is the total weight of all hydrophilic substances (ii) plus the total weight of all humectants and excluding the weight of all volatile cyclopropene compounds and excluding the weights of all molecular encapsulation agents, and T is the sum of H plus the total weight of all hydrophobic substances (i). In preferred embodiments, powder particles of volatile cyclopropene compound encapsulated in a molecular encapsulation agent meets one or more of the criteria for a hydrophilic substance, but the quantity H does not include the weight of any volatile cyclopropene compound, and the quantity H does not include the weight of any molecular encapsulation agent. Preferably, HI is 0.35 or greater; more preferably 1 or greater; more preferably 3 or greater; more preferably 7 or greater. Preferably, HI is 16 or smaller; more preferably 13 or smaller.

Preferred compositions of the present invention contain one or more of the following combinations, in each case optionally with additional ingredients:

polyethylene glycol having number average molecular weight of more than 3,000 with potassium acetate and with microcrystalline wax;

hydrophilic copolymer of ethylene and vinyl acetate with polyethylene glycol having number average molecular weight of 3,000 or less and with stearic acid;

hydrophilic copolymer of ethylene and vinyl acetate with polyvinyl pyrrolidone and with polyethylene glycol having number average molecular weight of 3,000 or less and with stearic acid;

hydrophilic copolymer of ethylene and vinyl acetate with cellulose and with polyethylene glycol having number average molecular weight of 3,000 or less and with stearic acid;

polyethylene glycol having number average molecular weight of 3,000 or less with microcrystalline wax and with stearic acid; and

polyethylene glycol having number average molecular weight of more than 3,000 with cellulose and with potassium acetate and with microcrystalline wax.

The composition of the present invention may be made by any method. A preferred method is melt mixing. An example of a procedure for melt mixing is the following: some or all of the ingredients are placed in a vessel and heated to TMIX, which is a temperature that is above room temperature and that is above 25°C. TMIX is chosen so that one or more of the ingredients in the vessel is a liquid. The mixture of ingredients is stirred, additional ingredients (if any) are added, the mixture of ingredients is further stirred, and then the mixture of ingredients is allowed to cool to room temperature, which will be between 0°C and 30°C. Two or more of the ingredients may be combined with each other prior to being combined with other ingredients during a melt-mixing process. It is contemplated that as the mixture of ingredients cools from TMIX to room temperature, the matrix as described herein will form a continuous phase and that any ingredients that are not dissolved in the matrix will be dispersed therein.

Preferably, every hydrophilic substance (ii) is either dissolved in the matrix or is dispersed in the matrix. Preferably, no hydrophilic substance or any mixture of hydrophilic substances forms a separated phase with any dimension of 0.5 mm or larger. Preferably, no hydrophilic substance or any mixture of hydrophilic substances forms a channel through the matrix. Preferably, the composition of the present invention does not have the form of two co-continuous phases in which one of the phases is a hydrophobic substance and the other phase is a hydrophilic substance.

One ingredient in the composition of the present invention is one or more encapsulation complex of a volatile cyclopropene compound encapsulated in a molecular encapsulation complex. Such an encapsulation complex is often supplied as a powder, known herein as an "EC" powder. EC powders may optionally contain additional compounds (i.e., in addition to the volatile cyclopropene compound and the molecular encapsulation complex), such as, for example, one or more of the following: water, one or more monosaccharide (such as, for example, dextrose), one or more disaccharide, one or more amino acid salt, or one or more adjuvant selected from vegetable oils, waxes, cellulose derivatives, carbohydrates, plasticizers, or surfactants. Preferably the amount of volatile cyclopropene in the EC powder, by weight based on the weight of the ED powder, is 0.01% or more; more preferably 0.03% or more; more preferably 0.1% or more. Preferably the amount of volatile cyclopropene in the EC powder, by weight based on the weight of the ED powder, is 10% or less; more preferably 6% or less.

Preferably the amount of cyclopropene compound in the composition of the present invention is, by weight based on the weight of the composition, 0.01% or more; more preferably 0.03% or more; more preferably 0.1% or more. Preferably the amount of cyclopropene compound in the composition of the present invention is, by weight based on the weight of the composition, 1% or less; more preferably 0.5% or less; more preferably 0.3% or less.

The composition of the present invention may be used in any of a wide variety of ways. In preferred embodiments, a solid object is made that contains the composition of the present invention. For example, a portion of the composition may be formed into solid mass. Some convenient shapes for such a solid mass include, for example, disks, pellets, films, rectangular solids, and other shapes.

For another example, a layer of the composition may be formed on a substrate. That layer may be applied to the substrate by applying a liquid as a coating by any coating method, such as, for example, brushing, spreading, spraying, metering, or other method. In some embodiments, the liquid that is applied to the substrate may have the composition of the present invention dissolved or dispersed in a liquid solvent or other liquid carrier; the solvent or other liquid carrier evaporates, leaving behind the composition of the present invention. In preferred embodiments, the liquid that is applied to the substrate is the fluid formed by heating the composition of the present invention. It is contemplated that the layer will be solidified or allowed to solidify by cooling or by removal of solvent or other liquid carrier or both.

In embodiments in which a layer of the composition of the present invention is formed on a substrate, regardless of the method by which that layer is formed, the composition may be put to use either by removing the layer from the substrate or by leaving the layer adhered to the substrate.

In preferred embodiments, the solids content of the composition of the present invention is 80% or higher; more preferred is 90% or higher.

In preferred uses of the present invention, a solid object that contains the composition of the present invention is inside a container. That solid object may be part of, or may be adhered or attached to an inside surface of a container, or that solid object may be present unattached inside the container. Preferably the container contains one or more plant or plant part; more preferably the container contains one or more fruit or vegetable after it has been harvested. It is contemplated that the plants or plant parts, through evaporation or metabolic processes or both, will provide water vapor to the atmosphere inside the container. It is contemplated that such water vapor will promote the release of volatile cyclopropene compound from the composition of the present invention.

Among embodiments that are inside a container that also contains one or more plant or plant part, it is useful to characterize the amount of cyclopropene compound (in micrograms) per liter of volume of the container. Preferably, that amount is 1 microgram/l or more; more preferably 2 micrograms/l or more; more preferably 5 micrograms/l or more; more preferably 10 micrograms/l or more. Preferably, that amount is 500 microgram/l or less; more preferably 250 micrograms/l or less; more preferably 100 micrograms/l or less; more preferably 50 micrograms/l or less.

In preferred embodiments, the container is not sealed and it is possible for gases to move in and out of the container. The container, the composition of the present invention, and the plants or plant parts may all be chosen so that a desired concentration of volatile cyclopropene is maintained in the atmosphere of the container, as volatile cyclopropene leaves the composition of the present invention to enter the atmosphere inside the container and as volatile cyclopropene then move out of the container into the atmosphere outside the container. The preferred concentration of volatile cyclopropene compound in the atmosphere of the container is 50 ppb (parts per billion, volume of volatile cyclopropene gas on volume of atmosphere) or more; more preferred is 100 ppb or more; more preferred is 200 ppb or more. The preferred concentration of volatile cyclopropene compound in the atmosphere of the container is 5,000 ppb or less; more preferred is 2,000 ppb or less; more preferred is 1,000 ppb or less.

Exposing plants or plant parts to cyclopropene compound may be beneficial. Cyclopropene compounds may block the effects of ethylene on plants or plant parts. Many of the processes that are triggered by ethylene are not beneficial to harvested plants or plant parts that are in storage. In some embodiments, for example, exposure to cyclopropene may lengthen the useful life of harvested fruits or vegetables.

Preferably, a container that contains one or more fruit or vegetable and that contains the composition of the present invention will be placed in an environment in which the container and its contents will be kept at a temperature higher than 0°C and lower than 25°C. More preferably, the temperature will be 1°C or higher, more preferably 2°C or

higher. More preferably, the temperature will be 10°C or lower; more preferably 5°C or lower.

Preferably, the composition of the present invention will remain inside a container together with one or more plant or plant part for 1 day or more; more preferably 2 days or more; more preferably 4 days or more. Preferably, the composition of the present invention will remain inside a container together with one or more plant or plant part for 30 days or less; more preferably 14 days or less.

The following are examples of the present invention.

The following abbreviations are used herein:

| Abbreviation | Meaning |
|---|---|
| aCD | alpha-cyclodextrin |
| 1-MCP | 1-methylcyclopropene |

These materials were used in the following Examples:

| Material | Description | Supplier |
|---|---|---|
| PEG 8000 | polyethylene glycol, molecular weight of 8,000 | |
| XL-PVP | crosslinked poly(vinyl pyrrolidone) | |
| cellulose | | |
| PEG 1000 | polyethylene glycol, molecular weight of 1,000 | |
| wax | microcrystalline wax | |
| Ethylene/vinyl acetate polymer | Elvax™ 250, ethylene/vinyl acetate copolymer, 28% vinyl acetate by weight | du Pont, Inc. |
| stearic acid | double pressed stearic acid | |
| KOAc | potassium acetate | |
| Mol. Sieve | Molecular Sieve | |
| $CaCO_3$ | | |
| Dispersant | | |
| Powder 1 | Powder containing an encapsulation complex of 1-MCP in aCD. Concentration of 1-MCP was about 5% by weight based on the weight of Powder1. | |

In the Examples below, "molten mixtures" were prepared as follows. The materials listed were mixed by mechanical stirring at about 80°C

Release of 1-MCP was measured as follows.

Experiments were performed in bottles that were sealed with a septum. Periodically, a sample of air was removed from the space inside the bottle by a needle pushed through the septum. That sample of air was analyzed for concentration of 1-MCP by gas chromatography. The amount of 1-MCP in the air could then be characterized as a percentage of the 1-MCP that had been placed into the bottle ("%release"). The %release was monitored as a function of time ("# days" means herein "number of days").

Comparative Example A:

20 mg of Powder1 and 3 ml of water were mixed in a bottle with a septum cap at 22 °C. All of the 1-MCP was released quickly.

| Results | |
|---|---|
| time | % release |
| 30 min. | 100% |

Comparative Example B

20 mg of Powder1 was suspended in a paper mesh over 3 ml of water in a bottle with a septum cap at 22 °C. All of the 1-MCP was released relatively quickly.

| Results | |
|---|---|
| **# days** | **% release** |
| 1 | 95% |

Comparative Example CA

| Materials | | |
|---|---|---|
| **wt %** | **Material** | **Function** |
| 1.0% | dispersant | |
| 60.0% | wax | meltable hydrophobic |
| 15.0% | potassium acetate | |
| 6.3% | molecular sieves | |
| 11.5% | calcium carbonate | |
| 6.3% | Powder1 | |

The mixture of materials was coated onto a paper in a 0.5 mm thick layer and cooled. A piece of the cooled material was removed and suspended in the neck of a bottle with a septum cap at 22 °C. Three ml of water was placed in the bottom of the bottle. No meltable hydrophilic compound was included. Release of 1-MCP was undesirably slow.

| Results at 22°C | |
|---|---|
| **# days** | **% release** |
| 1 | 2% |
| 3.5 | 9% |

Comparative Example CB

The experiment of Comparative Example CA was repeated, except that the temperature of the bottle during the experiment was 4°C. Release of 1-MCP was undesirably slow.

| Results at 4°C | |
|---|---|
| **# days** | **% release** |
| 1 | 1% |
| 3.5 | 2% |

Comparative Example D

| Materials | | |
|---|---|---|
| **wt %** | **Material** | **Function** |
| 34.0% | potassium acetate | humectant |
| 33.0% | molecular sieves | water adsorptive |
| 33.0% | Powder1 | |

About 300 mg of the mixture of materials was placed in an open plastic weigh boat and suspended in the neck

of a 2200 ml bottle containing 30 ml of water and equipped with a septum cap at 22 °C. No solid hydrophobic substance was used. Release of 1-MCP was undesirably fast.

| Results | |
|---|---|
| **# days** | **% release** |
| 0.05 | 50% |
| 0.11 | 61% |
| 0.22 | 78% |

Comparative Example E

| Materials | | |
|---|---|---|
| **wt %** | **Material** | **Function** |
| 90.0% | PEG 1000 | humectant |
| 10.0% | Powder1 | |

A molten mixture of the materials was prepared and cooled. About 500 mg of the mixture was placed in an open plastic weigh boat and suspended in the neck of a 2200 ml bottle containing 30 ml of water and equipped with a septum cap at 22 °C. No meltable hydrophobic substance was used. Release of 1-MCP was undesirably fast.

| Results | |
|---|---|
| **# days** | **% release** |
| 0.04 | 7% |
| 0.15 | 20% |
| 1.01 | 64% |

Example 1A

| | Materials | |
|---|---|---|
| **wt %** | **Material** | **Function** |
| 23.4% | PEG 8000 | meltable hydrophilic |
| 2.3% | dispersant | |
| 39.1% | wax | meltable hydrophobic |
| 15.6% | potassium acetate | humectant |
| 11.7% | molecular sieves | water-adsorptive |
| 7.8% | Powder 1 | |

A molten mixture of the materials was coated in a 0.5 mm thick layer and cooled. A piece of the cooled substrate was removed from the substrate and suspended in the neck of a bottle with a septum cap at 22 °C. Three ml of water was placed in the bottom of the bottle. Release was desirably gradual.

| Results | |
|---|---|
| **# days** | **% release** |
| 1 | 33% |
| 3.5 | 92% |

Example 1B

The experiment of Example 1A was repeated, except that the temperature of the bottle during the experiment was 4°C. Release was desirably gradual.

| Results | |
|---|---|
| **# days** | **% release** |
| # days | % release |
| 1 | 14% |
| 3.5 | 47% |
| 6 | 54% |

Example 2

| Materials | | |
|---|---|---|
| **wt %** | **Material** | **Function** |
| 15.0% | polyethylene glycol 1000 | meltable hydrophilic and humectant |
| 10.0% | ethylene-vinyl acetate polymer | hydrophobic |
| 40.0% | double pressed stearic acid | meltable hydrophobic |
| 5.0% | molecular sieves | water-adsorptive |
| 25.0% | calcium carbonate | thickener |
| 5.0% | Powder1 | |

A molten mixture of the materials was melted, mixed, cooled, and 200 mg was pressed into a disk 12.7 mm in diameter. The disk was suspended in the neck of a bottle with a septum cap at 5 °C. Three ml of water was placed in the bottom of the bottle. Release was desirably gradual.

| Results | |
|---|---|
| **# days** | **% release** |
| 1 | 20% |
| 4 | 60% |
| 8 | 70% |

Example 3

| Materials | | |
|---|---|---|
| **wt %** | **Material** | **Function** |
| | PEG 1000 | humectant |
| 11.1% | ethylene-vinyl acetate polymer | meltable hydrophilic |
| 44.4% | double pressed stearic acid | meltable hydrophobic |
| 22.2% | molecular sieves | water-adsorptive |
| 5.6% | Powder1 | |

A molten mixture of the materials was melted, mixed, cooled, and 200 mg was pressed into a disk 12.7 mm in diameter. The disk was suspended in the neck of a bottle with a septum cap at 5 °C. Three ml of water was placed in the bottom of the bottle. Release was desirably gradual.

| Results | |
|---|---|
| **# days** | **% release** |
| 1 | 15% |
| 4 | 35% |
| 8 | 60% |

Example 4

| Materials | | |
|---|---|---|
| **wt %** | **Material** | **Function** |
| 18.1% | polyethylene glycol 1000 | meltable hydrophilic and humectant |
| 12.0% | ethylene-vinyl acetate polymer | meltable hydrophilic |
| 12.0% | crosslinked polyvinyl pyrrolidone | hydrophilic |
| 48.2% | double pressed stearic acid | meltable hydrophobic |
| 4.8% | molecular sieves | water-adsorptive |
| 4.8% | Powder 1 | |

A mixture of the materials was melted, mixed, cooled, and 200 mg was pressed into a disk 12.7 mm in diameter. The disk was suspended in the neck of a bottle with a septum cap at 5 °C. Three ml of water was placed in the bottom of the bottle. Release was desirably gradual.

| Results | |
|---|---|
| **# days** | **% release** |
| 1 | 10% |
| 4 | 40% |
| 8 | 72% |

Example 5

| Materials | | |
|---|---|---|
| **wt %** | **Material** | **Function** |
| 18.1% | polyethylene glycol 1000 | meltable hydrophilic and humectant |
| 12.0% | ethylene-vinyl acetate polymer | meltable hydrophobic |
| 12.0% | cellulose | non-meltable hydrophilic |
| 48.2% | double pressed stearic acid | hydrophobic |
| 4.8% | molecular sieves | water-adsorptive |
| 4.8% | Powder1 | |

A mixture of the materials was melted, mixed, cooled, and 200 mg was pressed into a disk 12.7 mm in diameter. The disk was suspended in the neck of a bottle with a septum cap at 5 °C. Three ml of water was placed in the bottom of the bottle. Release was desirably gradual.

| Results | |
|---|---|
| **# days** | **% release** |
| 1 | 10% |
| 4 | 30% |
| 8 | 59% |

Example 6

| Materials | | |
|---|---|---|
| **wt %** | **Material** | **Function** |
| 11.7% | polyethylene glycol 1000 | meltable hydrophilic and humectant |
| 0.8% | dispersant | |
| 35.2% | wax | meltable hydrophobic |
| 27.3% | double pressed stearic acid | meltable hydrophobic |
| 19.5% | molecular sieves | water-adsorptive |
| 3.9% | Powder 1 | |

A mixture of the materials was melted, and the molten mixture was coated in a 0.5 mm thick layer onto paper, cooled, and a piece was suspended in the neck of a bottle with a septum cap at 4 °C. Three ml of water was placed in the bottom of the bottle. Release was desirably gradual.

Results

| time (days) | % release |
|---|---|
| 1 | 18% |
| 4 | 36% |
| 8 | 65% |

Example 7

Materials:

| wt % | Material | Function |
|---|---|---|
| 33.5% | PEG 8000 | meltable hydrophilic |
| 2.5% | dispersant | |
| 34.6% | wax | meltable hydrophobic |
| 5.6% | molecular sieves | water-adsorptive |
| 11.2% | potassium acetate | humectant |
| 5.6% | cellulose | thickener and non-meltable hydrophilic |
| 5.6% | Powder1 | |

Mixture was melted, mixed, cooled, and a 0.5 mm thick layer was coated onto paper. A portion was suspended in the neck of a bottle with a septum cap at 4 °C. Three ml of water was placed in the bottom of the bottle. Release was desirably gradual.

Results:

| time (days) | % release |
|---|---|
| 0.75 | 10% |
| 1.75 | 20% |
| 2.75 | 30% |
| 5.75 | 40% |

## Claims

**1.** A composition comprising

(a) a meltable solid matrix comprising

(i) one or more hydrophobic substance, and
(ii) one or more hydrophilic substance, and

(b) distributed in said matrix, one or more encapsulation complex of a volatile cyclopropene compound encapsulated in a molecular encapsulation agent.

**2.** The composition of claim 1, wherein there is distributed in said matrix one or more water-adsorptive substance.

**3.** The composition of claim 1, wherein there is distributed in said matrix one or more humectant, one or more deliquescent substance, or a mixture thereof.

**4.** The composition of claim 1, wherein said matrix additionally comprises one or more dispersant.

**5.** The composition of claim 1, wherein the volatile cyclopropene compound is 1-methylcyclopropene.

**6.** The composition of claim 1, wherein the molecular encapsulation agent is alpha-cyclodextrin.

**7.** The composition of claim 1, wherein the amount of volatile cyclopropene compound is 0.01% to 1% by weight, based on the weight of said composition.

**8.** A method of treating plants or plant parts comprising placing said plants or plant parts and the composition of claim 1 into a container and allowing said plants or plant parts and said composition of claim 1 to remain together in said container for 1 day or longer.

**9.** The method of claim 8, wherein said volatile cyclopropene compound is 1-methylcyclopropne, and wherein said molecular encapsulation agent is alpha-cyclodextrin.

**10.** The method of claim 9, wherein the amount of 1-methylcyclopropene placed into said container is 1 microgram per liter to 500 microgram per liter.

Europäisches Patentamt
European Patent Office
Office européen des brevets

# EUROPEAN SEARCH REPORT

Application Number
EP 12 15 9293

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 6 426 319 B1 (KOSTANSEK EDWARD CHARLES [US]) 30 July 2002 (2002-07-30)<br>* the whole document *<br>----- | 1-10 | INV.<br>A01N27/00<br>A01N25/18<br>A01N25/08 |
| X | EP 1 340 425 A1 (ROHM & HAAS [US])<br>3 September 2003 (2003-09-03)<br>* the whole document *<br>----- | 1-10 | |
| Y | WO 99/39574 A2 (SOUTHWEST RES INST [US]; BERNARD TECHNOLOGIES INC [US])<br>12 August 1999 (1999-08-12)<br>* the whole document *<br>----- | 1-10 | |
| Y | US 5 914 120 A (WELLINGHOFF STEPHEN T [US] ET AL) 22 June 1999 (1999-06-22)<br>* the whole document *<br>----- | 1-10 | |

TECHNICAL FIELDS SEARCHED (IPC)

A01N

The present search report has been drawn up for all claims

1

| Place of search | Date of completion of the search | Examiner |
|---|---|---|
| Munich | 20 August 2012 | Bertrand, Franck |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 9293

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2012

| Patent document cited in search report | Publication date | Patent family member(s) | Publication date |
|---|---|---|---|
| US 6426319 B1 | 30-07-2002 | AR 030755 A1 | 03-09-2003 |
| | | AU 783225 B2 | 06-10-2005 |
| | | AU 7215501 A | 11-04-2002 |
| | | BR 0104286 A | 04-06-2002 |
| | | CA 2357892 A1 | 29-03-2002 |
| | | CN 1346594 A | 01-05-2002 |
| | | DE 60100143 D1 | 30-04-2003 |
| | | DE 60100143 T2 | 27-11-2003 |
| | | EP 1192858 A1 | 03-04-2002 |
| | | ES 2194818 T3 | 01-12-2003 |
| | | IL 145476 A | 05-07-2006 |
| | | JP 4884619 B2 | 29-02-2012 |
| | | JP 2002179508 A | 26-06-2002 |
| | | KR 20020025728 A | 04-04-2002 |
| | | MX PA01009745 A | 15-04-2002 |
| | | NZ 514236 A | 31-01-2003 |
| | | TW 589140 B | 01-06-2004 |
| | | US 2002061822 A1 | 23-05-2002 |
| EP 1340425 A1 | 03-09-2003 | AT 294502 T | 15-05-2005 |
| | | AU 2003200594 A1 | 11-09-2003 |
| | | BR 0300235 A | 03-08-2004 |
| | | CA 2419341 A1 | 27-08-2003 |
| | | CN 1440649 A | 10-09-2003 |
| | | DE 60300588 D1 | 09-06-2005 |
| | | DE 60300588 T2 | 19-01-2006 |
| | | DK 1340425 T3 | 29-08-2005 |
| | | EP 1340425 A1 | 03-09-2003 |
| | | ES 2242143 T3 | 01-11-2005 |
| | | JP 3937168 B2 | 27-06-2007 |
| | | JP 2003286101 A | 07-10-2003 |
| | | MX PA03001082 A | 06-12-2004 |
| | | NZ 524289 A | 30-07-2004 |
| | | PT 1340425 E | 30-09-2005 |
| WO 9939574 A2 | 12-08-1999 | AU 757684 B2 | 06-03-2003 |
| | | AU 2571199 A | 23-08-1999 |
| | | CA 2320804 A1 | 12-08-1999 |
| | | CN 1313770 A | 19-09-2001 |
| | | EP 1071435 A2 | 31-01-2001 |
| | | HK 1038883 A1 | 28-01-2005 |
| | | JP 4083983 B2 | 30-04-2008 |
| | | JP 2002501872 A | 22-01-2002 |
| | | NZ 505857 A | 19-12-2003 |
| | | US 6605304 B1 | 12-08-2003 |
| | | WO 9939574 A2 | 12-08-1999 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 12 15 9293

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2012

| Patent document cited in search report | Publication date | Patent family member(s) | Publication date |
|---|---|---|---|
| US 5914120 A | 22-06-1999 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6426319 B
- US 20050288189 A

### Non-patent literature cited in the description

- **FW BILLMEYER, JR.** Textbook of Polymer Science. 1971